# EUROPÄISCHE PATENTSCHRIFT

(11) **EP 1 481 633 B1**
(45) Veröffentlichungstag und Bekanntmachung des Hinweises auf die Patenterteilung: **02.05.2007**
(21) Anmeldenummer: 04011103.1
(22) Anmeldetag: 10.05.2004
(51) Int. Cl.: A61B 5/00

(54) **Gerät zum Erfassen wenigstens einer Substanz in einem Stoffwechselprozess eines Lebewesens**
Apparatus for detecting a substance of metabolism of a living body
Dispositif pour la détection d'une substance du métabolisme d'un être vivant

(30) Priorität: 30.05.2003 DE 10324600
(43) Veröffentlichungstag der Anmeldung: 01.12.2004
(73) Patentinhaber: SIEMENS AKTIENGESELLSCHAFT, 80333 München (DE)
(72) Erfinder: Abraham-Fuchs, Klaus, 91058 Erlangen (DE); Gareus, Ralph, Dr., 91301 Forchheim (DE); Hengerer, Arne, Dr., 91054 Erlangen (DE); Sieber, Heike, 96047 Bamberg (DE)

(56) Entgegenhaltungen:
- EP-A- 0 770 349
- DE-A- 10 109 539
- US-A- 4 407 295
- US-A- 5 628 982
- US-A- 6 122 536
- US-A- 6 149 889
- US-A1- 2002 091 324
- US-B1- 6 207 136

## Beschreibung

Die Erfindung betrifft ein gerät zum Erfassen wenigstens einer Substanz in einem Stoffwechselprozess eines Lebewesens.

Es sind Geräte zur Messung des Zeitverlaufs der Konzentration eines metabolischen Parameters, insbesondere tragbare Sensorgeräte, beispielsweise Blutglukosemessgeräte oder Geräte zur Messung der Sauerstoffsättigung des Hämoglobins, bekannt, bei denen spezifische Glukose- bzw. Hämoglobindioxid-Sensoren direkt die Zielsubstanz messen. Beispielsweise aus der DE 195 40 456 A1 ist ferner eine künstliche Pankreas bekannt, umfassend einen auf Auswerten eines physikalischen Parameters von Blut basierenden Glukosesensor, ein Insulinreservoir, eine Insulinabgabeeinrichtung und einen Mikroprozessor als Regler, wobei der Glukosesensor quasi kontinuierlich die Glukosekonzentration im Blut ermittelt und die Insulinabgabeeinrichtung dazu veranlasst, aus dem Insulinreservoir bedarfsgerecht, d.h. in Abhängigkeit von der Glukosekonzentration, Insulin abzugeben. Dabei ist die künstliche Pankreas voll implantierbar oder extern am Patienten anordenbar, wobei der Glukosesensor beispielsweise in einem Gefäß des Patienten liegt, während die Insulinabgabe subkutan erfolgt. Der Glukosesensor bestimmt dabei die Blutglukosekonzentration und ihre Dynamik, also den zeitlichen Verlauf, quasi kontinuierlich und gibt diese Information an den Regler weiter.

Ein Gerät gemäß dem Oberbegriff von Anspruch 1 ist aus der DE 101 09 539 A1 bekannt.

Eine Aufgabe der Erfindung ist es, ein verbessertes Gerät zum Erfassen wenigstens einer Substanz in einem Stoffwechselprozess eines Lebewesens zu schaffen, mit dem unter anderem auch eine schwer erfassbare Substanz in einem Lebewesen detektierbar ist.

Die Aufgabe wird durch den Gegenstand des Anspruchs 1 gelöst. Vorteilhafte Ausgestaltungen sind in den Unteransprüchen beschrieben.

Dabei wird zum Monitoring des Zeitverlaufs eines Stoffwechselprozesses ein molekulares, insbesondere biomolekulares Kontrastmittel verabreicht, beispielsweise in den Körper eines Lebewesens injiziert, oral aufgenommen, usw., welches spezifisch an einen Stoffwechselbaustein, beispielsweise ein umsetzendes Molekül, insbesondere ein Enzym, bzw. ein Stoffwechselprodukt, beispielsweise ein Substrat, der interessierenden Stoffwechselkette bindet bzw. mittels einer biochemischen Reaktion mit diesem Stoffwechselbaustein bzw. Stoffwechselprodukt in einen signalgebenden Zustand aktiviert wird. Dadurch sind insbesondere Stoffwechselbausteine bzw. Stoffwechselprodukte detektierbar, deren direkte Erfassung, insbesondere durch eine Haut des Lebewesens hindurch gar nicht oder nur mit großem Aufwand möglich ist. Dabei ist das spezifisch gebundene Molekül bzw. das aktivierte Kontrastmittel mittels eines Detektors nachweisbar, wobei der Detektor in ein entsprechendes Gerät integriert ist, welches über einen längeren Zeitraum am Körper des Lebewesens getragen werden kann. Das Gerät weist optional Mittel zur Signalauswertung und/oder Signalübertragung auf.

Es sind dabei zahlreiche Kontrastmittel bekannt, die mittels molekularem Engineering hochspezifisch an Zielmoleküle, beispielsweise Proteine, binden und anschließend mittels einer daran gekoppelten signalgebenden Substanz ein außerhalb des Körpers messbares Signal aussenden, so dass das Vorhandensein der Zielmoleküle und gegebenenfalls deren Konzentration nachgewiesen werden kann. Beispiele derartiger Kontrastmittel sind in der US 6,083,486 beschrieben, wobei diese aus mehreren funktionalen Komponenten bestehen, deren Alternativen in der Spalte 1, Zeile 61 bis Spalte 3, Zeile 29 der US 6,083,486 aufgelistet sind. Gemäß dem Stand der Technik werden diese biomolekularen Kontrastmittel, insbesondere Fluoreszenzkontrastmittel, zur optischen Bildgebung und beim Einsatz in der Pharmaforschung mit Kleintieren, beispielsweise Mäusen, verwendet. Damit wird vorrangig die räumliche Verteilung eines Zielmoleküls im Körper dargestellt, die beispielsweise zur Diagnose und Lokalisierung eines Tumors dient. Da aber mit solchen Kontrastmitteln beispielsweise Proteine, DNA- und/oder RNA-Abschnitte detektiert werden können, welche Ausgangspunkt bzw. Reagenz einer metabolischen Prozesskette sind, sind solche Kontrastmittel auch geeignet, einen interessierenden metabolischen Prozess hochspezifisch zu charakterisieren. Biomolekulare Kontrastmittel, wie beispielsweise in der vorgenannten US 6,083,486 beschrieben, können auch so konstruiert werden, dass zwischen aktiven und inaktiven Proteinvarianten unterschieden werden kann oder dass die Aktivität von Enzymen selbst oder von durch die Enzyme erzeugten Abbauprodukten gemessen werden kann. Gemäß der Erfindung kann damit in hochspezifischer Weise der Umsatz eines für einen biologischen Stoffwechselprozess charakteristischen Reagenzoder Abbauprodukts gemessen und somit auf die Stoffwechselrate geschlossen werden.

Dabei ist die Konzentration eines Moleküls aus einer Stoffwechselkette über einen längeren Zeitraum messbar, womit die von medizinischem Interesse geprägten Fragestellungen beantwortbar sind, ob ein bestimmter metabolischer Prozess, beispielsweise ein Stoffwechselvorgang, der charakteristisch für einen Tumor ist, oder ein Abbauvorgang beispielsweise in der Leber überhaupt stattfindet, in welchen zeitlichen Abständen, ausgedrückt in Minuten, Stunden oder Tagen, der Stoffwechselvorgang stattfindet, mit welcher Intensität ein Stoffwechselvorgang stattfindet und/oder ob die Intensität derzeit zunehmend, abnehmend oder gleichbleibend ist.

Für das Erfassen des Kontrastmittels kann dabei davon ausgegangen werden, dass der Ort bekannt ist, an dem die Konzentration des interessierenden metabolischen Markers in einer aussagekräftigen Konzentration vorhanden ist. So werden beispielsweise viele metabolische Marker in nachweisbarer Konzentration in der Blutbahn transportiert und sind somit an jeder Stelle des Blutgefäßsystems messbar. Andere geeignete Messorte können auf der Körperoberfläche direkt über Körperorganen, beispielsweise der Leber, der Niere oder Lymphknoten, oder direkt über Läsionen, beispielsweise Tumore und Operationswunden, usw., sein. An dieser als geeignet identifizierten Stelle wird beispielsweise ein portables, am Körper tragbares Gerät aufgesetzt, das einen Detektor enthält, welcher die vom molekularen Kontrastmittel ausgesendeten Signale im Zeitverlauf, beispielsweise als Fluoreszenzsignal, Magnetfeld oder radioaktive Strahlung messen und im Zeitverlauf aufzeichnen kann. Beispielsweise bezogen auf die Kontrastmittel der US 6,083,486, deren Wellenlängen in der Tabelle 1 der US 6,083,486 angegeben sind, muss der Detektor geeignet sein, die vom Fluoreszenzkontrastmittel emittierte Wellenlänge zu detektieren. Dabei wird in vorteilhafter Weise unter Verwendung eines vorgeschalteten optischen Filters selektiv detektiert.

Weitere Vorteile, Merkmale und Einzelheiten der Erfindung ergeben sich aus den im Folgenden beschriebenen Ausführungsbeispielen anhand der Figuren. Dabei zeigen:
Figur 1 ein Gerät zum fortlaufenden Erfassen wenigstens einer durch ein molekulares Kontrastmittel hervorgehobenen Substanz in einem Stoffwechselprozess eines Lebewesens,
Figur 2 Ausführungsformen des Geräts als Armband und Implantat, wobei letztere Ausführungsform nicht Bestandteil der Erfindung ist, und
Figur 3 Ausführungsformen des Geräts als Gehörganggerät und Ohrklipp.

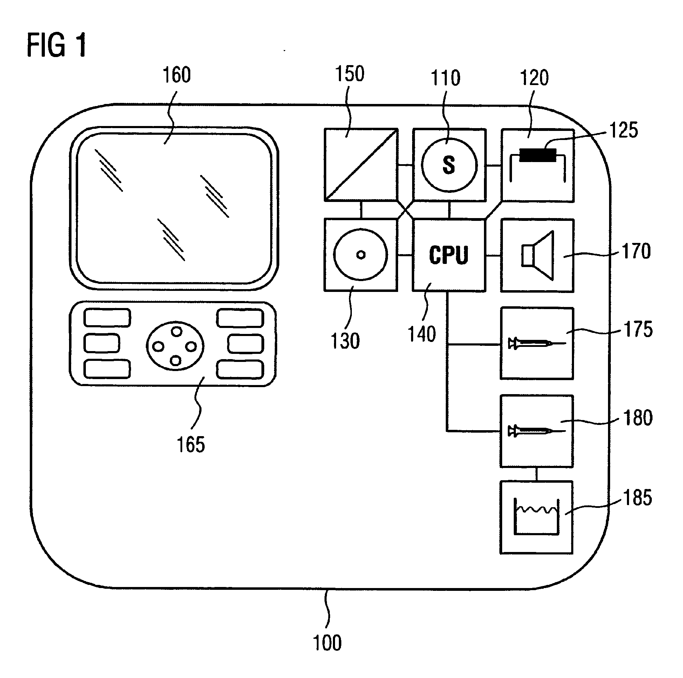

Die Figur 1 zeigt als ein Ausführungsbeispiel der Erfindung ein Gerät 100 zum fortlaufenden Erfassen wenigstens einer Substanz in einem Stoffwechselprozess eines Lebewesens, die durch ein molekulares Kontrastmittel hervorgehoben ist, mit einer Detektoreinheit 110 zum Detektieren des dem Lebewesen verabreichten molekularen Kontrastmittels. In einer Ausführungsform der Erfindung wird ein Fluoreszenzkontrastmittel verwendet und dementsprechend ist die im Gerät 100 integrierte Detektoreinheit 110 für dieses Kontrastmittel als ein Detektor zur Messung von Fluoreszenzsignalen ausgebildet, umfassend beispielsweise einen CCD-Chip.

In einer weiteren Ausführungsform der Erfindung ist das Kontrastmittel derart gestaltet, dass es mittels einer in das Gerät 100 integrierten Anreichereinheit 120 in räumlicher Nähe der Detektoreinheit 110 angereichert werden kann. Dies kann beispielsweise dadurch erreicht werden, dass das Kontrastmittel magnetische Eigenschaften besitzt, beispielsweise Eisenoxidpartikel umfasst, und die Anreichereinheit 120 einen Magneten umfasst, so dass die magnetischen Kontrastmittelmoleküle zum Magneten hingezogen werden bzw. bei Transport durch das Gefäßsystem in der Nähe des Magneten im Blutgefäß teilweise immobilisiert werden. Der Magnet kann als ein Elektromagnet 125 ausgestaltet sein, so dass die Immobilisierung der Moleküle an- und abgeschaltet werden kann. Durch ein derartiges Vorgehen können die Moleküle in der Nähe der Detektoreinheit 110 angereichert und somit ein verstärktes Messsignal gewonnen werden.

Das Gerät 100 umfasst weiterhin eine Speichereinheit 130, in der der Zeitverlauf des von der Detektoreinheit 110 aufgenommenen Messsignals gespeichert wird. Des Weiteren ist wenigstens eine Schnittstelleneinheit 150 zur drahtlosen und/oder kabelgebundenen Übertragung dieser Signale an einen Empfänger zum Auswerten und/oder Darstellen der Signale vorgesehen. In einer Ausführungsform ist das Messsignal auch ohne Zwischenspeicherung mittels drahtloser Übertragung an einen Empfänger übertragbar.

Des Weiteren umfasst das Gerät 100 eine Anzeigeeinheit 160 zur alphanumerischen und/oder graphischen Darstellung der Stärke des Stoffwechselprozesses und/oder zur Anzeige des Trends des Stoffwechselprozesses im Sinne von zunehmend, abnehmend oder gleichbleibend, eine Alarmgebereinheit 170, die bei Über- oder Unterschreiten eines voreingestellten Schwellwerts des Messsignals aktiv wird, eine Eingabeeinheit 165, beispielsweise eine Tastatur zum Einstellen von Geräteoptionen oder Eingabe von Daten, beispielsweise eines Schwellwertes, eine Prozessoreinheit 140 zum Auswerten der Messdaten und/oder eine Dosiereinheit 175 für Medikamente.

In einer Ausführungsform sind dabei wenigstens die Detektoreinheit 110 und/oder die Prozessoreinheit 140 derart hergerichtet, dass nur die Anwesenheit oder Abwesenheit des Messsignals bzw. des über dem Schwellwert einer Hintergrundaktivität bzw. eines Grundrauschens liegenden Messsignals erfasst wird und daraus auf das Vorliegen oder Nichtvorliegen des beobachteten Stoffwechselprozesses geschlossen wird. In einer anderen Ausführung, bei der davon ausgegangen wird, dass die Intensität des gemessenen Signals proportional zur Konzentration des die Zielmoleküle hervorhebenden Anteils des Kontrastmittels, damit zur Konzentration des Zielmoleküls und damit zur Stärke des Stoffwechselvorgangs ist, ist insbesondere die Detektoreinheit 110 für ein intensitätssensitives Erfassen hergerichtet.

Des Weiteren ist das Gerät 100 in einer Ausführungsform derart hergerichtet, dass ein Zeitpunkt der Gabe des Kontrastmittels in das Gerät 100 eingegeben und gespeichert werden kann, um diesen Zeitpunkt bei der Auswertung und gegebenenfalls Darstellung der Signale berücksichtigen zu können. Ebenso kann in einer Ausführungsform die Menge des verabreichten Kontrastmittels eingegeben werden.

Ferner umfasst das Gerät 100 eine Reservoireinheit 185 für das Kontrastmittel und eine Injektionseinheit 180 für das Kontrastmittel, mittels derer Teile des in der Reservoireinheit 185 befindlichen Kontrastmittels in dosierter Menge auf Kommando oder in voreinstellbaren Zeitintervallen in das Lebewesen injiziert werden können.

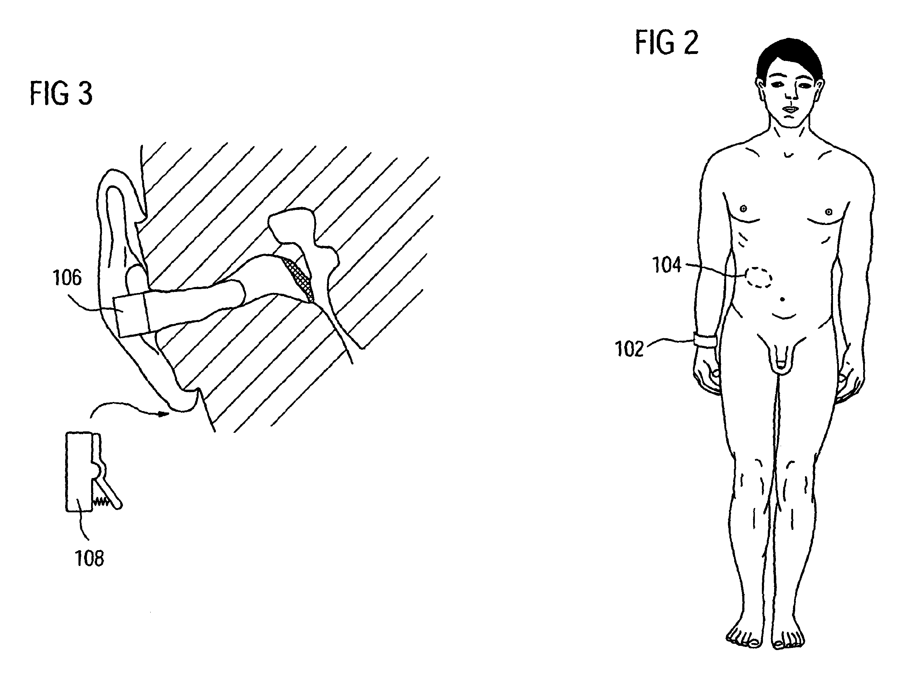

Die Figur 2 zeigt als Ausführungsbeispiel der Erfindung eine Realisierungen des Geräts 100 in der Ausbildung als Armband 102. Es ist auch eine nicht zur Erfindung gehörende Realisierung als Implantat 104 gezeigt. Dabei ist beim Armband 102 beispielsweise die Detektoreinheit 110 derart in das Armband 102 integriert, dass die Detektoreinheit 110 in geeigneter Weise über einem Hauptblutgefäß des Handgelenks platziert wird.

Die Figur 3 zeigt schließlich als Ausführungsbeispiele der Erfindung ein Schnittbild im Bereich des linken Gehörorgans des Patienten aus Figur 2 mit Realisierungen des Geräts 100 als Gehörganggerät 106 und Ohrklipp 108. Dabei ist das Gehörganggerät 106 derart konstruiert, dass mit der Detektoreinheit 110 Signale aus dem Gefäßsystem des Gehörgangs aufnehmbar sind. Beim Ohrklipp 108 ist die Detektoreinheit 110 derart integriert, dass die Detektoreinheit 110 in geeigneter Weise über dem Gefäßsystem des Ohrläppchens platziert wird.

In Ausführungsformen der Erfindungen beinhalten die am Körper tragbaren Geräteausführungen 102 bis 108 nicht alle bei der Figur 1 beschriebenen Einheiten 110 bis 185 und/oder wenigstens zwei der Einheiten 110 bis 185 sind auf wenigstens zwei der Geräteausführungen 102 bis 108 verteilt angeordnet. So kann beispielsweise die Detektoreinheit 110 im Ohrklipp 108 und die Anzeige- und Eingabeeinheit 160 und 165 im Armband 102 untergebracht sein.

## Patentansprüche

1. Gerät zum Erfassen wenigstens einer Substanz in einem Stoffwechselprozess eines Lebewesens umfassend einen Detektor, der derart ausgebildet ist, einen die Substanz hervorhebenden Anteil eines dem Lebewesen verabreichten molekularen Kontrastmittels an wenigstens einer Stelle des Lebewesens zeitlich fortlaufend zu erfassen, und eine Speichereinheit, in der ein Zeitverlauf eines von dem Detektor aufgenommenen Messsignals speicherbar ist, **dadurch gekennzeichnet, dass** das Gerät als ein am Körper des Lebewesens tragbares Gerät ausgebildet ist.

2. Gerät nach Anspruch 1, **dadurch gekennzeichnet, dass** Mittel zum Aufzeichnen von Ergebnissen des zeitlich fortlaufenden Erfassens vorhanden sind.

3. Gerät nach Anspruch 1 oder 2, **dadurch gekennzeichnet, dass** das Erfassen in einer Reihe von zeitlich mit einem vorgebbaren Zeitabstand aufeinander folgenden Zeitpunkten durchgeführt wird

4. Gerät nach Anspruch 3, **dadurch gekennzeichnet, dass** der Zeitabstand einige Sekunden, Minuten oder Stunden umfasst.

5. Gerät nach einem der obigen Ansprüche, **dadurch gekennzeichnet, dass** das Kontrastmittel ein Fluoreszenzkontrastmittel ist.

6. Gerät nach einem der obigen Ansprüche, **dadurch gekennzeichnet, dass** das Kontrastmittel ein über sein Magnetfeld detektierbares Kontrastmittel sind.

7. Gerät nach einem der obigen Ansprüche, **dadurch gekennzeichnet, dass** das Kontrastmittel ein über seine radioaktive Strahlung detektierbares Kontrastmittel sind.

8. Gerät nach einem der obigen Ansprüche, **dadurch gekennzeichnet, dass** der Detektor Mittel zum optischen Erfassen eines die Substanz hervorhebenden Anteils des Kontrastmittels umfasst.

9. Gerät nach Anspruch 8, **dadurch gekennzeichnet, dass** die Mittel einen CCD-Chip umfassen.

10. Gerät nach einem der obigen Ansprüche, **dadurch gekennzeichnet, dass** wenigstens ein Teil des Geräts in ein Armband integriert ist.

11. Gerät nach einem der obigen Ansprüche, **dadurch gekennzeichnet, dass** wenigstens ein Teil des Geräts in einen Ohrklipp integriert ist.

12. Gerät nach einem der obigen Ansprüche, **dadurch gekennzeichnet, dass** wenigstens ein Teil des Geräts in einem Gehörgang des Lebewesens tragbar ausgebildet ist.

13. Gerät nach einem der obigen Ansprüche, **dadurch gekennzeichnet, dass** das Gerät eine alphanumerische und/oder graphische Anzeigeeinheit umfasst.

14. Gerät nach einem der obigen Ansprüche, **dadurch gekennzeichnet, dass** das Gerät eine Alarmgebereinheit umfasst.

15. Gerät nach einem der obigen Ansprüche, **dadurch gekennzeichnet, dass** das Gerät eine Eingabeeinheit zum Eingeben von Daten umfasst.

16. Gerät nach einem der obigen Ansprüche, **dadurch gekennzeichnet, dass** das Gerät eine Dosiereinheit für wenigstens ein Medikament umfasst.

17. Gerät nach einem der obigen Ansprüche, **dadurch gekennzeichnet, dass** das Gerät eine Injektionseinheit für das Kontrastmittel umfasst.

18. Gerät nach einem der obigen Ansprüche, **dadurch gekennzeichnet, dass** das Gerät eine Schnittstelleneinheit zum drahtlosen und/oder kabelgebundenen Übertragen von Daten umfasst.

19. Gerät nach einem der obigen Ansprüche, **dadurch gekennzeichnet, dass** das Gerät Mittel zum Anreichern des Kontrastmittels umfasst.

20. Gerät nach einem der obigen Ansprüche, **dadurch gekennzeichnet, dass** bei einem magnetisch anreicherbaren Kontrastmittel die Mittel einen wenigstens ein- und ausschaltbar ausgebildeten Magneten umfassen.

21. Gerät nach einem der obigen Ansprüche, **dadurch gekennzeichnet, dass** das Gerät eine Auswerteeinheit zum Ermitteln einer Aktivität und/oder einer Rate des Stoffwechsels umfasst.

22. Gerät nach Anspruch 21, **dadurch gekennzeichnet, dass** die Aktivität eine Rate des Stoffwechsels, eine als zunehmend, abnehmend oder konstant klassifizierbare Tendenz einer Intensität des Stoffwechsels, eine Zeitdauer von Periodizitätszyklen und/oder eine Häufigkeit, Zeitdauer und/oder einen Zeitabstand eines Auftretens bei einem aperiodisch auftretenden Stoffwechselprozess umfasst.

23. Gerät nach einem der obigen Ansprüche, **dadurch gekennzeichnet, dass** Mittel zum Berechen einer Rate des Stoffwechsels vorhanden sind, wobei die Rate aus einem Ergebnis des Erfassens als Umsatzmenge in Gewicht oder Volumen der Substanz pro Zeiteinheit mittels eines Models des Stoffwechsels berechnet wird.

24. Gerät nach Anspruch 24, **dadurch gekennzeichnet, dass** bei den Mitteln zum Berechen der Rate das Model als einzig unbekannten variablen Parameter das Ergebnis des Erfassens neben weiteren bekannten Parametern des Models beinhaltet.

## Claims

1. Device for detecting at least one substance in a metabolic process of a living being, that highlights the substance and a memory unit, in which a variation over time of a measurement signal recorded by the detector can be stored, **characterized in that** the device is formed as a device which can be carried on the body of the living being. a molecular contrast agent which is administered to the living being comprising a detector formed in such a way as to detect continuously over time at at least one location of the living being a component of ... . .

2. Device according to claim 1, **characterized in that** means are present for recording results of the continuous detection over time.

3. Device according to claim 1 or 2, **characterized in that** the detection is carried out at a series of points in time following one after the other in time with a predeterminable time interval.

4. Device according to claim 3, **characterized in that** the time interval comprising several seconds, minutes or hours.

5. Device according to one of the preceding claims, **characterized in that** the contrast agent is a fluorescent contrast agent.

6. Device according to one of the preceding claims, **characterized in that** the contrast agent is a contrast agent which can be detected on the basis of its magnetic field.

7. Device according to one of the preceding claims, **characterized in that** the contrast agent is a contrast agent which can be detected on the basis of its radioactive radiation.

8. Device according to one of the preceding claims, **characterized in that** the detector comprises means for optically detecting a component of the contrast agent that highlights the substance.

9. Device according to claim 8, **characterized in that** the means comprises a CCD chip.

10. Device according to one of the preceding claims, **characterized in that** at least part of the device is integrated in an arm band.

11. Device according to one of the preceding claims, **characterized in that** at least part of the device is integrated in an ear clip.

12. Device according to one of the preceding claims, **characterized in that** at least part of the device is formed such that it can be carried in an auditory canal of the living being.

13. Device according to one of the preceding claims, **characterized in that** the device comprises an alphanumeric and/or graphic display unit.

14. Device according to one of the preceding claims, **characterized in that** the device comprises an alarm transmitter unit.

15. Device according to one of the preceding claims, **characterized in that** the device comprises an input unit for entering data.

16. Device according to one of the preceding claims, **characterized in that** the device comprises a dosing unit for at least one medicament.

17. Device according to one of the preceding claims, **characterized in that** the device comprises an injection unit for the contrast agent.

18. Device according to one of the preceding claims, **characterized in that** the device comprises an interface unit for the wireless and/or cable-bound transmission of data.

19. Device according to one of the preceding claims, **characterized in that** the device comprises means for enriching the contrast agent.

20. Device according to one of the preceding claims, **characterized in that** the means comprises in the case of a contrast agent which can be magnetically enriched a magnet formed such that it can at least be switched on and off.

21. Device according to one of the preceding claims, **characterized in that** the device comprises an evaluation unit for determining an activity and/or a rate of the metabolism.

22. Device according to claim 21, **characterized in that** the activity comprises a rate of the metabolism, a tendency of an intensity of the metabolism that can be classified as increasing, decreasing or constant, a time duration of periodicity cycles and/or a frequency, time duration and/or a time interval of an occurrence in an aperiodically occurring metabolic process.

23. Device according to one of the preceding claims, **characterized in that** means are present for calculating a rate of the metabolism, the rate being calculated by means of a model of the metabolism from a result of the detection as an amount in weight or volume of the substance transformed per unit of time.

24. Device according to claim 24, **characterized in that** with the means for calculating the rate the model comprises the result of the detection as the only unknown variable parameter, along with further known parameters of the model.

## Revendications

1. Appareil de détection d'au moins une substance dans un métabolisme d'un être vivant, comprenant un détecteur conçu pour détecter continuellement dans le temps, en au moins un endroit de l'être vivant, une proportion d'un agent de contraste moléculaire administré à l'être vivant et mettant en évidence la substance, et une unité de mémoire dans laquelle peut être mémorisée une variation en fonction du temps d'un signal de mesure enregistré par le détecteur, **caractérisé en ce que** l'appareil est constitué sous la forme d'un appareil pouvant être porté sur le corps de l'être vivant.

2. Appareil suivant la revendication 1, **caractérisé en ce qu'**il y a des moyens d'enregistrement des résultats de la détection continue dans le temps.

3. Appareil suivant la revendication 1 ou 2, **caractérisé en ce que** la détection est effectuée en une série d'instants se succédant dans le temps à un intervalle de temps pouvant être prescrit.

4. Appareil suivant la revendication 3, **caractérisé en ce que** l'intervalle de temps comprend quelques secondes, minutes ou heures.

5. Appareil suivant l'une des revendications ci-dessus, **caractérisé en ce que** l'agent de contraste est un agent de contraste fluorescent.

6. Appareil suivant l'une des revendications ci-dessus, **caractérisé en ce que** l'agent de contraste est un agent de contraste pouvant être détecté par son champ magnétique.

7. Appareil suivant l'une des revendications ci-dessus, **caractérisé en ce que** l'agent de contraste est un agent de contraste pouvant être détecté par son rayonnement radioactif.

8. Appareil suivant l'une des revendications ci-dessus, **caractérisé en ce que** le détecteur comprend des moyens de détection optiques d'une proportion de l'agent de contraste qui met en évidence la substance.

9. Appareil suivant la revendication 8, **caractérisé en ce que** les moyens comprennent une puce CCD.

10. Appareil suivant l'une des revendications ci-dessus, **caractérisé en ce qu'**au moins une partie de l'appareil est intégrée à un bracelet.

11. Appareil suivant l'une des revendications ci-dessus, **caractérisé en ce qu'**au moins une partie de l'appareil est intégrée à un clip auriculaire.

12. Appareil suivant l'une des revendications ci-dessus, **caractérisé en ce qu'**au moins une partie de l'appareil est constituée de manière à pouvoir être portée dans le conduit auditif de l'être vivant.

13. Appareil suivant l'une des revendications ci-dessus, **caractérisé en ce que** l'appareil comprend une unité d'affichage alphanumérique et/ou graphique.

14. Appareil suivant l'une des revendications ci-dessus, **caractérisé en ce que** l'appareil comprend une unité pour donner l'alerte.

15. Appareil suivant l'une des revendications ci-dessus, **caractérisé en ce que** l'appareil comprend une unité d'entrée de données.

16. Appareil suivant l'une des revendications ci-dessus, **caractérisé en ce que** l'appareil comprend une unité d'addition dosée d'au moins un médicament.

17. Appareil suivant l'une des revendications ci-dessus, **caractérisé en ce que** l'appareil comprend une unité d'injection de l'agent de contraste.

18. Appareil suivant l'une des revendications ci-dessus, **caractérisé en ce que** l'appareil comprend une unité d'interface pour la transmission de données sans fil et/ou par câble.

19. Appareil suivant l'une des revendications ci-dessus, **caractérisé en ce que** l'appareil comprend des moyens d'enrichissement en l'agent de contraste.

20. Appareil suivant l'une des revendications ci-dessus, **caractérisé en ce que**, pour un agent de contraste que l'on peut enrichir par voie magnétique, les moyens comprennent un aimant constitué de manière à pouvoir être au moins mis en circuit et hors circuit.

21. Appareil suivant l'une des revendications ci-dessus, **caractérisé en ce que** l'appareil comprend une unité d'exploitation pour déterminer une activité et/ou un taux du métabolite.

22. Appareil suivant la revendication 21, **caractérisé en ce que** l'activité comprend un taux du métabolisme, une tendance pouvant être classée en croissante, décroissante ou constante d'une intensité du métabolisme, une durée de cycles de périodicité et/ou une fréquence, une durée et/ou un laps de temps d'une apparition pour un métabolisme se produisant de façon apériodique.

23. Appareil suivant l'une des revendications ci-dessus, **caractérisé en ce qu'**il y a des moyens de calcul d'un taux du métabolisme, le taux étant calculé à partir d'un résultat de la détection sous la forme d'une quantité de conversion en poids ou en volume de la substance par unité de temps au moyen d'un modèle du métabolisme.

24. Appareil suivant la revendication 23, **caractérisé en ce que**, dans les moyens de calcul du taux, le modèle comprend, comme paramètre variable unique inconnu, le résultat de la détection, outre d'autres paramètres connus du modèle.
